# FASCICULE DE BREVET EUROPEEN

(11) **EP 1 535 925 B1**
(45) Date de publication et mention de la délivrance du brevet: **27.05.2009**
(21) Numéro de dépôt: 04292616.2
(22) Date de dépôt: 04.11.2004
(51) Int. Cl.: C07F 7/08, C08K 5/541, A61K 8/58, A61Q 17/04

(54) **Méthyltrialkylsilanes à fonction 4,4-diarylbutadiènene et compositions cosmétiques ou dermatologiques photoprotectrices les contenant**
4,4-Diarylbutadien-funktionalisierte Methyltrialkylsilane und diese enthaltende kosmetische oder dermatologische Lichtschutzzusammensetzungen
4,4-Diarylbutadiene functionalized methyltrialkylsilanes and cosmetic or photoprotective dermatological compositions containing them

(30) Priorité: 25.11.2003 FR 0350899
(43) Date de publication de la demande: 01.06.2005
(73) Titulaire: L'ORÉAL, 75008 Paris (FR)
(72) Inventeur: Richard, Hervé, 93420 Villepinte (FR)
(74) Mandataire: Miszputen, Laurent

(56) Documents cités:
- EP-A- 0 916 335
- DE-A- 19 820 116
- DATABASE CAPLUS CHEMICAL ABSTRACTS SERVICE, COLUMBUS, OHIO, US; XP002295387 extrait de STN Database accession no. 1995:780700 & JP 07 157489 A (SHINETSU CHEMICAL INDUSTRY CO LTD) 20 juin 1995 (1995-06-20)
- DATABASE CAPLUS CHEMICAL ABSTRACTS SERVICE, COLUMBUS, OHIO, US; XP002295388 extrait de STN Database accession no. 1995:462856 & JP 07 017983 A (SHISEIDO CO LTD) 20 janvier 1995 (1995-01-20)

## Description

L'invention concerne de nouveaux méthyltrialkylsilanes à fonction 4,4-diarylbutadiène.

L'invention concerne l'utilisation de ces méthyltrialkylsilanes à fonction 4,4-diarylbutadiène dans la préparation de compositions cosmétiques ou dermatologiques comme agent de filtration des rayons UV de longueurs d'onde comprises entre 320 et 400 nm.

L'invention concerne des compositions cosmétiques ou dermatologiques photoprotectrices , caractérisées par le fait qu'elle contiennent dans un support cosmétiquement acceptable au moins l'un de ces méthyltrialkylsilanes à fonction 4,4-diarylbutadiène.

On sait que les radiations de longueurs d'onde comprises entre 280 nm et 400 nm permettent le brunissement de l'épiderme humain, et que les radiations de longueurs d'onde comprises entre 280 et 320 nm connues sous la dénomination de radiations UV-B, provoquent des érythèmes et des brûlures cutanées qui peuvent nuire au développement du bronzage naturel. Pour ces raisons ainsi que pour des raisons esthétiques, il existe une demande croissante de moyens de contrôle de ce bronzage naturel. Il convient donc de filtrer ce rayonnement UV-B.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible et/ou continuellement exposée au rayonnement solaire. Les rayons UV-A entraînent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photoallergiques. Ainsi, pour des raisons esthétiques et cosmétiques telles que la conservation de l'élasticité naturelle de la peau, de plus en plus de personnes désirent contrôler l'effet des rayons UV-A sur leur peau. Il est donc souhaitable de filtrer aussi le rayonnement UV-A.

Il existe aujourd'hui un besoin de plus en plus important de rechercher des composés aromatiques efficaces pour la filtration des radiations UV-A et de préférence dans la gamme allant de 340 à 400 nm (gamme dite des UV-A longs ou UVA-I). Outre leur bonne efficacité de photoprotection dans cette gamme, les filtres UV-A recherchés doivent présenter de bonnes propriétés cosmétiques, une bonne solubilité dans les solvants usuels et en particulier dans les corps gras tels que les huiles et les graisses, une bonne résistance à l'eau et à la transpiration (rémanence) ainsi qu'une photostabilité satisfaisante.

Il existe actuellement sur le marché des filtres UV un nombre limité de composés organiques efficaces vis à vis des rayons UV-A et notamment des UV-A longs.

A cet égard, une famille de filtres UV-A particulièrement efficace dans la gamme des UV-A est actuellement constituée par les dérivés du dibenzoylméthane, et notamment le 4-ter-butyl-4'-méthoxydibenzoyl méthane, qui présentent en effet un fort pouvoir d'absorption intrinsèque. Ces dérivés du dibenzoylméthane, qui sont maintenant des produits bien connus en soi à titre de filtres actifs dans les UV-A, sont notamment décrits dans les demandes de brevets français FR-A-2326405 et FR-A-2440933, ainsi que dans la demande de brevet européen EP-A-0114607 ; le 4-ter-butyl-4'-méthoxydibenzoyl méthane est par ailleurs actuellement proposé à la vente sous la dénomination commerciale de "PARSOL 1789" par la Société HOFFMANN LAROCHE.

Malheureusement, il se trouve que les dérivés du dibenzoylméthane sont des produits relativement photosensibles au rayonnement UV-A, c'est-à-dire, plus précisément, qu'ils présentent une fâcheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que des applications répétées à intervalles de temps réguliers et rapprochés doivent être effectuées par l'utilisateur pour obtenir une protection efficace de la peau contre les rayons UV.

Une autre difficulté, indépendante de celle évoquée ci-avant, rencontrée avec les dérivés de dibenzoylméthane est qu'il s'agit de filtres lipophiles présentant la particularité mais aussi le désavantage d'être solides à température ambiante. De ce fait, leur utilisation dans une composition cosmétique antisolaire implique certaines contraintes au niveau de leur formulation et de leur mise en oeuvre, en particulier lorsqu'il s'agit de trouver des solvants permettant de les solubiliser correctement, seuls ou conjointement avec d'autres filtres.
On connaît dans la demande EP842938 comme filtres UVA lipophiles des composés siloxaniques ou silaniques à groupements flavones. On connaît également dans la demande US20010053856 comme filtres lipophiles UVA et UVB des composés siloxaniques ou silaniques à groupements benz-x-azole.

Il existe donc un besoin de rechercher de nouvelles familles de composés aromatiques qui soient efficaces en matière de filtration dans la gamme des UV-A et notamment celle des UVA longs mais qui soient photostables, présentent également à la fois une bonne solubilité dans les solvants usuels, de bonnes propriétés cosmétiques ainsi qu'une bonne résistance à l'eau et à la transpiration (rémanence).

La demanderesse a découvert de manière surprenante une nouvelle famille de méthyltrialkylsilanes à fonction 4,4-diarylbutadiène permettant d'atteindre ces objectifs.

Un objet de la présente invention consiste en une nouvelle famille de composés méthyltrialkylsilanes à fonction 4,4-diarylbutadiène de formule générale (1) que l'on définira en détail ci-dessous.

L'invention concerne l'utilisation d'au moins un méthyltrialkylsilane à fonction 4,4-diarylbutadiène de formule générale (1) que l'on définira en détail ci-dessous dans la préparation des compositions cosmétiques ou dermatologiques comme agent de filtration des rayons UV de longueurs d'onde comprises entre 320 et 400 nm.

L'invention concerne des compositions photoprotectrices L'invention concerne de nouveaux méthyltrialkylsilanes à fonction 4,4-diarylbutadiène.

L'invention concerne l'utilisation de ces méthyltrialkylsilanes à fonction 4,4-diarylbutadiène dans la préparation de compositions cosmétiques ou dermatologiques comme agent de filtration des rayons UV de longueurs d'onde comprises entre 320 et 400 nm.
L'invention concerne des compositions cosmétiques ou dermatologiques photoprotectrices caractérisées par le fait qu'elle contiennent dans un support cosmétiquement acceptable au moins un méthyltrialkylsilane à fonction 4,4-diarylbutadiène de formule générale (1) que l'on définira en détail ci-dessous.
Les méthyltrialkylsilanes à fonction 4,4-diarylbutadiène, conformes à l'invention sont sous forme isolée ou de mélange et correspondent à la formule générale (1) suivante : 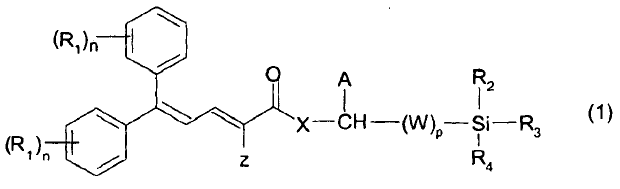 dans laquelle :
- les radicaux R₁, égaux ou différents désignent un radical hydroxyle ; un radical alkyle en C₁-C₁₀, linéaire ou ramifié, saturé ou insaturé ; un radical alcoxy en C₁-C₁₀, linéaire ou ramifié ; 2 groupes R₁ adjacents pouvant former ensemble un cycle alkylène dioxy en C₁-C₃ ;
- les radicaux R₂, R₃ et R₄ identiques ou différents, représentent un radical alkyle en C₁-C₁₀, linéaire ou ramifié, saturé ou insaturé, pouvant comporter un ou plusieurs atomes d'halogène (par exemple Cl, Br, F) ; un radical phényle,
- p est égal à 0 ou 1.
- A désigne hydrogène ; un radical alkyle en C₁-C₈, linéaire ou ramifié, saturé ou insaturé; un radical phényle ; le groupe Si(CH₃)₃, sous réserve que lorsque A est Si(CH₃)₃, alors p = 0 et R₂, R₃ et R₄ sont méthyle,
- n est un nombre entier allant de 0 à 2,
- W est un radical alkylène en C₁-C₈, linéaire ou ramifié, saturé ou insaturé, éventuellement substitué par un radical hydroxyle,
- Z désigne hydrogène ; -(C=O)OR₅, -(C=O)R₆, -(C=O)NR₇R₈, -SO₂R₉, -CN ou -(C=O)XCHA(W)ₚSiR₂R₃R₄,
- le radical R₅ désigne hydrogène ; un radical alkyle en C₁-C₂₀, linéaire ou ramifié, saturé
ou insaturé,
- le radical R₆ désigne un radical alkyle en C₁-C₂₀, linéaire ou ramifié, éventuellement cyclique ; un aryle en C₆-C₁₂,
- les radicaux R₇, R₈, identiques ou différents représentent hydrogène ; un alkyle en C₁-C₂₀, linéaire ou ramifié,
- X désigne -O- ou -NR₇-,
- le radical R₉désigne un radical alkyle en C₁-C₂₀, linéaire ou ramifié ; un aryle en C₆-C₁₂.

Bien que dans la formule (1) ci-dessus, seuls soient représentés les isomères dans lesquels le substituant Z est en position cis par rapport au substituant diaryle, cette formule doit être comprise comme englobant également l'isomère trans correspondant.

Dans la formule (1) ci-dessus, les radicaux alkyles peuvent être linéaires ou ramifiés, saturés ou insaturés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Le radical alkyle particulièrement préféré est le radical méthyle.

Dans la formule (1) ci-dessus, les radicaux alcoxy peuvent être linéaires ou ramifiés et choisis notamment au sein des radicaux méthoxy, éthoxy, n-propoxy, isopropoxy, n-butoxy et isobutoxy. Le radical alcoxy particulièrement préféré est le radical méthoxy.

Dans la formule (1) ci-dessus, les radicaux aryle sont de préférence phényle.

Les méthyltrialkylsilanes à fonction 4,4-diarylbutadiène de formule (1) présentant de préférence au moins l'une, et encore plus préférentiellement l'ensemble, des caractéristiques suivantes :
- Z = -(C=O)OR₅, -CN ou (C=O)XCHA(W)ₚSiR₂R₃R₄,
- R₅ est méthyle ou éthyle,
- A est H,
- R₂ à R₄ désignent alkyle en C₁-C₄ et plus préférentiellement méthyle,
- n est 0,
- p est 0 ou 1,
- W est un radical alkylène en C₁-C₂

Parmi les méthyltrialkylsilanes à fonction 4,4-diarylbutadiène de formule (1) préférés, on peut citer les composés (A), (B), (C) de formules suivantes :

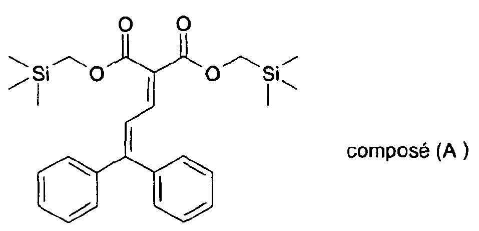

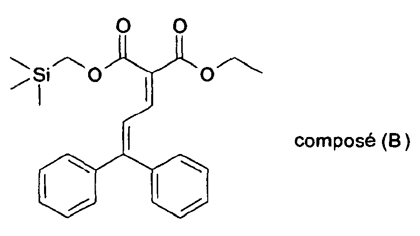

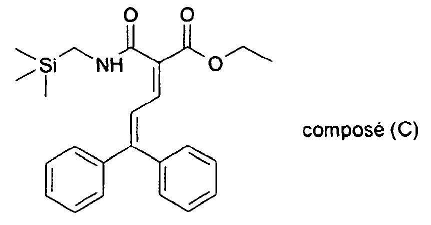

Une famille de méthyltrialkylsilanes à fonction 4,4-diarylbutadiène de formule (1) particulièrement préférés est constituée par ceux pour lesquels Z désigne -CN. Ces composés particuliers sont efficaces dans la gamme des rayons UVA longs (340-400nm).

Parmi ces composés, on utilisera de préférence ceux présentant au moins l'une des caractéristiques suivantes et encore plus préférentiellement l'ensemble des caractéristiques suivantes :
- A est H,
- R₂ à R₄ désignent alkyle en C₁-C₄ et plus préférentiellement méthyle,
- n est 0,
- p est 0 ou 1,
- W est un radical alkylène en C₁-C₂

Parmi ces composés , on utilisera encore plus particulièrement les composés (D), (E), (F) et (G) suivants :

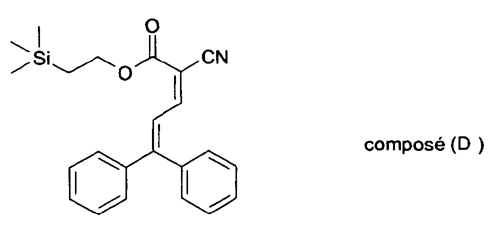

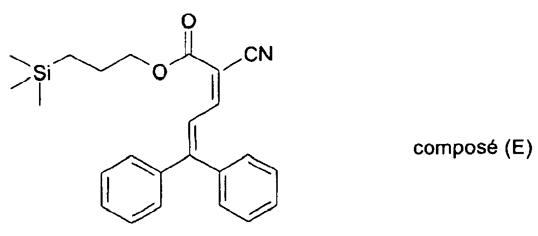

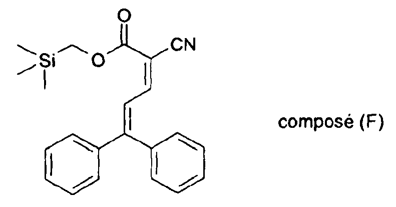

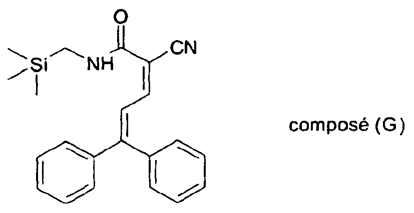

Les dérivés silaniques de formule (1) sont obtenus par condensation de Knoevenagel d'un dérivé de β-phényl cinnamaldéhyde de formule (2) sur un dérivé à méthylène activé de formule (3) comme décrit dans le brevet EP 0916 335 selon le Schéma (1) suivant : 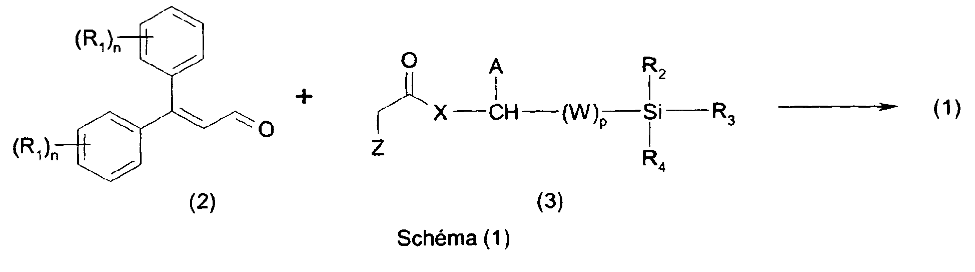 dans laquelle les radicaux R₁ à R₄, Z, X, A, W, n et p ont la même signification qu'à la formule (1).

Les dérivés de formule (3) peuvent être obtenus par une des trois voies décrites dans le schéma (2) suivant : 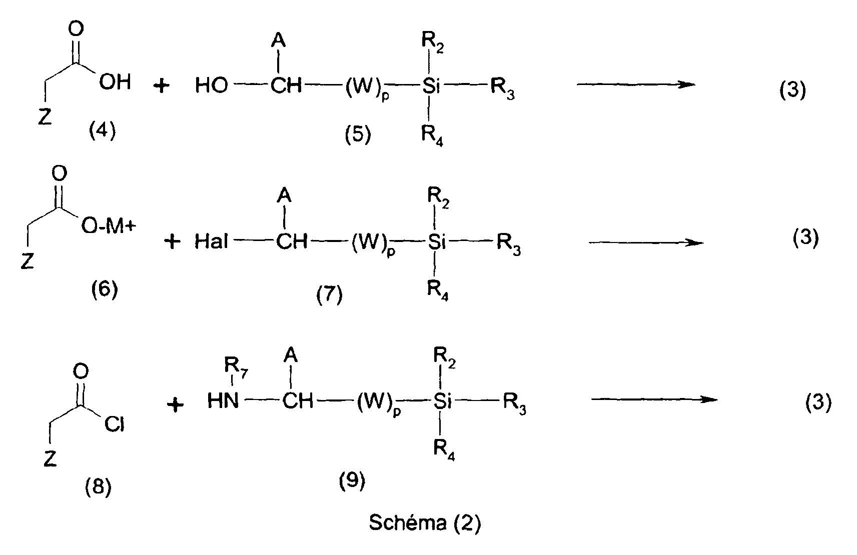 dans lequel Z, A, W, R₂ à R₄, R₇ et p ont les mêmes significations que celles indiquées ci-dessus pour la formule (1), Hal représente un halogène et plus particulièrement le chlore et M+ représente un métal alcalin et plus particulièrement le sodium ou le potassium.

Comme dérivés de formule (4), on peut citer l'acide cyanoacétique, le monoéthyl malonate (RN 1071-46-1) ou le mono-tert-butyl malonate qui sont des produits commerciaux.

Comme dérivés de formule (5), on peut citer l'hydroxyméthyltriméthylsilane (RN 3219-63-4) ou le 2-(triméthylsilyl)éthanol (RN 2916-68-9) qui sont des produits commerciaux.

Comme dérivés de formule (6), on peut citer le monoéthyl malonate de potassium (RN 6148-64-7) qui est un produit commercial.

Comme dérivés d'halogénure silanique de formule (7), on peut citer le chloropropyltriméthylsilane (RN 2344-83-4) ou le chlorométhyltriméthylsilane (RN 2344-80-1 ) vendus par la société WACKER.

Egalement on peut citer les produits commerciaux suivants : le iodométhyltriméthylsilane (RN 4206-67-1), le (chlorométhyl)diméthyléthylsilane (RN 3121-77-5), le (chlorométhyl)diméthyl-n-butylsilane (RN 3121-75-3), le (chlorométhyl)diméthylpentylsilane (RN 73013-39-5), le (chlorométhyl)dodécyldiméthylsilane (RN 70851-47-7), le (chlorométhyl)triéthylsilane (RN 757-34-6), le 2-choroéthyltriméthylsilane (RN 7787-87-3), le Bis(triméthylsilyl)méthylchloride (RN 5926-35-2), le (chlorométhyl)diméthyl-phénylsilane (RN 1833-51-8), le (chlorométhyl)diphénylméthylsilane (RN 18407-40-4) et le (triméthylsilylméthyl)diméthylchlorométhylsilane (RN 18306-73-5).

Comme dérivé de formule (8), on peut citer le chlorure d'acide du monoéthyl malonate ou le malonyl dichloride qui sont des produits commerciaux.

Comme dérivés de formule (9), on peut citer l'aminométhyltriméthylsilane (RN 18166-02-4) vendu par la société Gelest ainsi que la (phényl)(triméthylsilyl)méthylamine ou la Bis(triméthylsilyl)méthylamine (RN 134340-00-4).

Les composés de formule (I) sont généralement présents dans la composition de l'invention dans des proportions comprises entre 0,01 % et 20 % en poids, de préférence entre 0,1 % et 10 % en poids, par rapport au poids total de la composition.

Les compositions conformes à l'invention peuvent comporter en plus d'autres filtres UV organiques ou inorganiques complémentaires actifs dans l'UVA et/ou l'UVB hydrosolubles, liposolubles ou bien insolubles dans les solvants cosmétiques couramment utilisés.

Les filtres organiques complémentaires sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes non siliciés tels que décrits dans les demandes EP0967200, DE19746654, DE19755649, EP-A-1008586, EP1133980 et EP133981 et leurs mélanges.

Comme exemples de filtres organiques complémentaires, on peut citer ceux désignés ci-dessous sous leur nom INCl :

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP, Glyceryl PABA,
PEG-25 PABA vendu sous le nom « UVINUL P25 » par BASF,

### Dérivés salicyliques :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

### Dérivés du dibenzoylméthane :

Butyl Methoxydibenzoylmethane vendu notamment sous le nom commercial « PARSOL 1789 » par HOFFMANN LA ROCHE,
Isopropyl Dibenzoylmethane,

### Dérivés cinnamiques :

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
- Diisopropyl Methylcinnamate,
Glyceryl Ethylhexanoate Dimethoxycinnamate

### Dérivés de β,β'-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF, Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial « UVINUL DS-49» par BASF,
Benzophenone-12
2-(4-diethylamino-2-hydroxybenzoyl)-benzoate de n-hexyle.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK ,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

### Dérivés de la triazine :

Anisotriazine vendu sous le nom commercial «TINOSORB S » par CIBA GEIGY,
Ethylhexyl triazone vendu notamment sous le nom commercial «UVINUL T150 » par BASF,
2,4,6-tris-(4'-amino benzalmalonate de diisobutyle)-s- triazine,
Diethylhexyl Butamido Triazone vendu sous le nom commercial « UVASORB HEB » par SIGMA 3V,

### Dérivés du phenyl benzotriazole :

Drometrizole Trisiloxane vendu sous le nom « Silatrizole » par RHODIA CHIMIE , Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques :

Menthyl anthranilate vendu sous le nom commercial commercial « NEO HELIOPAN MA » par Haarmann et REIMER,

### Dérivés d'imidazolines :

Ethylhexyl Dimethoxybenzylidene Dioxoimidazoline Propionate,

### Dérivés du benzalmalonate :

Polyorganosiloxane à fonction benzalmalonate comme le produit Polysilicone-15 vendu sous la dénomination commerciale « PARSOL SLX » par HOFFMANN LA ROCHE

### Dérivés de 4,4-diarylbutadiène non siliciés :

1,1-dicarboxy (2,2'-diméthyl-propyl)-4,4-diphénylbutadiène

### Dérivés de benzoxazole :

2,4-bis-[5-1(diméthylpropyl)benzoxazol-2-yl-(4-phenyl)-imino]-6-(2-ethylhexyl)-imino-1,3,5-triazine vendu sous le nom Uvasorb K2A par Sigma 3V ;
et leurs mélanges.

Les filtres UV organiques complémentaires préférentiels sont choisis parmi
Ethylhexyl Salicylate,
Ethylhexyl Methoxycinnamate
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
la 2,4,6-tris-(4'-amino benzalmalonate de diisobutyle)-s-triazine Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine
et leurs mélanges.

Les filtres complémentaires inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium ou leurs mélanges qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les filtres UV complémentaires conformes à l'invention sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions cosmétiques selon l'invention peuvent contenir en outre des agents de bronzage et/ou de brunissage artificiel de la peau (agents autobronzants) tels que la dihydroxyacétone (DHA). Ils sont présents de préférence dans des quantités allant de 0,1 à 10% et plus préférentiellement de 0,2 à 8% en poids par rapport au poids total de la composition.

Les compositions conformes à la présente invention peuvent comprendre en outre des adjuvants cosmétiques classiques notamment choisis parmi les corps gras, les solvants organiques, les épaississants ioniques ou non ioniques, les adoucissants, les humectants, les antioxydants, les hydratants, les desquamants, les agents anti-radicalaires, les agents antipollution, les antibactériens, les agents anti-inflammatoires, les dépigmentants, les pro-pigmentants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les pigments, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C12-C15 vendu sous la dénomination commerciale « Finsolv TN » par la société WITCO, le palmitate d'octyle, le lanolate d'isopropyle, les triglycérides dont ceux des acides caprique/caprylique), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Les épaississants peuvent être choisis notamment parmi les polymères acryliques réticulés comme les Carbomer, les polymères réticulés acrylates/C₁₀-C₃₀alkylacrylates du type Pemulen ou le polyacrylate-3 vendu sous le nom VISCOPHOBE DB 1000 par Amerchol ; les polyacrylamides tels que l'émulsion polyacrylamide, C₁₃-C₁₄ isoparraffine et laureth-7 vendue sous le nom SEPIGEL 305 par SEPPIC, les homopolymères ou copolymères d'AMPS tel l'HOSTACERIN AMPS vendu par CLARIANT, les gommes de guar et celluloses modifiées ou non telles que la gomme de guar hydroxypropylée, la méthylhydroxyéthylcellulose et l'hydroxypropylméthyl cellulose, la gomme de xanthane, les silices nanométriques de type Aerosil.

Bien entendu, l'homme de l'art veillera à choisir le ou les éventuels composés complémentaires cités ci-dessus et/ou leurs quantités de manière telle que les propriétés avantageuses attachées intrinsèquement aux composés conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, d'une huile, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Lorsque la composition cosmétique selon l'invention est utilisée pour le soin de l'épiderme humain, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, d'huile solaire, de bâtonnet solide, de poudre, de mousse aérosol ou de spray.

Lorsque la composition cosmétique selon l'invention est utilisée pour le soin des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des ongles, des lèvres, des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

Un autre objet de l'invention est l'utilisation d'au moins un composé de formule (1) tel que définie ci-dessus dans une composition cosmétique comme agent de contrôle de la variation de la couleur de la peau due aux rayonnements UV-A.

Un autre objet de l'invention est l'utilisation d'au moins un composé de formule (1) tel que définie précédemment comme agent photostabilisant de polymères synthétiques tels que les matières plastiques ou de verres en particulier des verres de lunettes ou des lentilles de contact.

Des exemples concrets, mais nullement limitatifs, illustrant l'invention, vont maintenant être donnés.

### EXEMPLE 1 : Préparation de l'ester di-(triméthylsilanylméthyle) de l'acide 3,3-diphényl-2-propylidène) propanedioique :

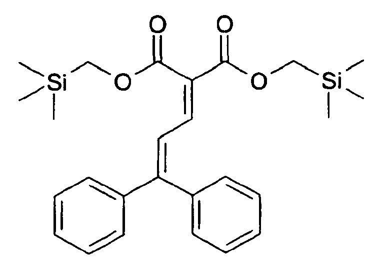

### a) Première étape : Préparation du malonate de triméthylsilanylméthyle :

Dans un réacteur surmonté par un Dean Stark, on porte au reflux pendant 4 heures l'acide malonique (20,7 g, 0,199 mole) et de l'hydroxyméthyltriméthylsilane (41,4 g, 0,398 mole) dans 150 ml de toluène en présence de 0,5 ml d'acide sulfurique concentré. L'eau formée est éliminée par azéotropie. La phase organique est lavée 2 fois à l'eau, séchée sur sulfate de sodium et concentrée sous pression réduite. Après distillation sous vide (Eb 90°C sous 0,02 hPa), on obtient 46,7 g (Rendement 85 %) du malonate de triméthylsilanylméthyle sous forme d'une huile incolore.

### b) Deuxième étape : préparation du composé de l'exemple 1 :

Dans un réacteur on solubilise le β-phényl cinnamaldéhyde (5 g, 0,024 mole) dans 100 ml d'isopropanol. On y ajoute 0,6 ml de pipéridine et le malonate de triméthylsilanylméthyle (7,1 g, 0,0258 mole). On chauffe le mélange réactionnel à 60°C pendant 2 heures. La solution est concentrée sous pression réduite. L'excès de malonate est éliminé par distillation sous un vide de 0,02 hPa. Après purification par chromatographie sur colonne de silice (éluant : Heptane puis Heptane/AcOEt 9 :1), on obtient les fractions propres du dérivé de l'exemple 1 (8,9 g, Rendement 80%) sous forme d'un liquide épais qui cristallise dans le temps :
- solide jaune pâle
- Pf 50°C
- UV (Ethanol)λₘₐₓ = 333 nm, εₘₐₓ = 28 610 , E_{1%} = 614.

### EXEMPLE 2 : Préparation du diester monotriméthylsilanylméthyle , monoéthyle de l'acide 3,3-diphényl-2-propylidène) propanedioique (composé B) :

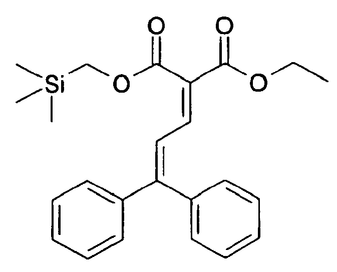

### a) Première étape : Préparation du malonate de monotriméthylsilanylméthyle monoéthyle :

Dans un réacteur, on porte à 70°C pendant 24 heures un mélange du sel potassique de l'acide monoéthyle malonique (17 g, 0,1 mole) et du chlorométhyltriméthylsilane (12,2 g, 0,1 mole) dans 50 ml de DMF. On verse dans 100 ml d'eau et extrait au dichlorométhane. La phase organique est lavée 2 fois à l'eau, séchée sur sulfate de sodium et concentrée sous pression réduite. On obtient 12 g d'une huile jaune pâle constituée du mélange de malonate de monotriméthylsilanylméthyle monoéthyle et de malonate de 2-(triméthylsilylméthyl) monotriméthylsilanylméthyle monoéthyle dans le rapport 4 :1. Ce mélange est utilisé tel quel dans l'étape suivante.

### b) Deuxième étape : préparation du composé de l'exemple 2 :

Dans un réacteur on solubilise le β-phényl cinnamaldéhyde (5 g, 0,024 mole) dans 200 ml d'isopropanol. On y ajoute 0,6 ml de pipéridine et le mélange de malonates de l'étape précédente (8,5 g, 0,0255 mole de 2/a). On chauffe le mélange réactionnel au reflux pendant 4 heures. Le solvant est évaporé sous pression réduite. On reprend l'huile obtenue dans le dichlorométhane et on lave à l'eau acidulée. La phase organique est lavée 2 fois à l'eau, séchée sur sulfate de sodium et concentrée sous pression réduite. Le malonate en excès et son impureté sont éliminées par distillation sous vide. Le résidu de distillation est chromatographié sur colonne de silice (éluant: Heptane puis Heptane/AcOEt 9:1) pour donner le composé de l'exemple 2 sous forme d'une huile incolore :
- CPG : présence des 2 isomères dans le rapport 50 :50.
- UV (Ethanol) λₘₐₓ = 334 nm, εₘₐₓ = 28 270 E_{1%} = 692.

### EXEMPLE 3 : Préparation de l'ester monotriméthylsilanylméthyle de l'acide 2-cyano-5,5-diphényl-2,4-diène pentanoïque (composé F) :

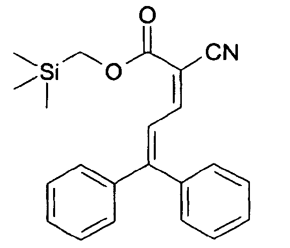

### a) Première étape : Préparation du cyanoacétate de triméthylsilanylméthyle :

Dans un réacteur surmonté par un Dean Stark, on porte au reflux pendant 4 heures de l' hydroxyméthyltriméthylsilane (3,8 g, 0,036 mole) et de l'acide cyanoacétique (3,1 g, 0,036 mole) dans 50 ml de toluène en présence d'une goutte d'acide sulfurique concentré. La phase organique est lavée 2 fois à l'eau et est séchée sur sulfate de sodium. Elle est concentrée sous pression réduite. Après distillation sous vide (Eb 80°C sous 0,4 hPa), on obtient 4,3 g (Rendement 69 %) du cyanoacétate de triméthylsilanylméthyle sous forme d'un liquide incolore utilisé tel quel dans l'étape suivante.

### b) Deuxième étape : préparation du composé de l'exemple 3 :

Dans un réacteur on solubilise le β-phényl cinnamaldéhyde (4,8 g, 0,023 mole) dans 400 ml d'isopropanol. On y ajoute 0,6 ml de pipéridine et le cyanoacétate de triméthylsilanylméthyle (4 g, 0,023 mole). On chauffe le mélange réactionnel au reflux pendant 2 heures. On ajoute de l'eau et extrait au dichlorométhane. La phase organique est lavée à l'eau acidulée puis séchée sur sulfate de sodium. Le solvant est évaporé sous pression réduite. On obtient une huile qui cristallise. Après recristallisation dans l'heptane, on obtient 6,5 g (Rendement 78 %) du dérivé de l'exemple 3 sous forme de cristaux jaunes pâles :
- Pf 84 °C
- UV (Ethanol)λₘₐₓ = 360 nm, εₘₐₓ = 28 880, E_{1%} = 800.

### EXEMPLE DE COMPOSITION A

| **Phase** | **Constituants** | **Concentration** **(g%)** |
|---|---|---|
| A | Mélange monostéarate de glycéryle / Stéarate de polyéthylène glycol (100 OE) (ARLACEL 165 - Uniquema) | 1 |
| | Acides gras d'origine végétale (STEARINE TP 1200 - Stearinerie Dubois) | 1.5 |
| | Diméthicone (DOW CORNING 200 FLUID 350 CS - Dow Corning) | 0.5 |
| | Alcool cétylique (LANETTE 16 - Cognis) | 0.5 |
| | Mélange cétylstéarylglucoside/ Alcool cétylstéarylique (MONTANOV 68 - Seppic) | 2 |
| | Benzoate d'alcools en C12-C15 FINSOLV TN - Witco | 10 |
| | Composé de l'exemple 1 | 5 |
| B | Glycérine (PRICERINE 9091 - Uniquema) | 5 |
| | Phosphate d'alcool hexadecylique, sel de potassium (AMPHISOL K - Roche Vitamins) | 1 |
| | EDTA | 0.1 |
| C | Gomme de xanthane (KELTROL T - CP KELCO) | 0.2 |
| | Copolymère réticulé Acrylates/C10-C30-alkylacrylate (PEMULEN TR-1 - Novéon) | 0.2 |
| | Isohexadécane (ISOHEXADECANE - BP) | 1 |
| | Triethanolamine | qs pH |
| | Conservateurs | qs |
| | Eau déminéralisée | qsp 100g |

### Protocole de fabrication :

On pèse la phase grasse (A) et on chauffe au bain marie à 70°C. On pèse la phase aqueuse (B) dans le bêcher final et on chauffe au bain marie à 70°C. Sous agitation Rotor/Stator type MORITZ, on disperse la phase grasse dans la phase aqueuse (environ 1000 tr/min.). On incorpore le mélange d'épaississants (C) et on laisse revenir à température ambiante sous agitation.Vers 30°C, on neutralise la formule et on conditionne.

## Revendications

1. Méthyltrialkylsilane à fonction 4,4-diarylbutadiène sous forme isolée ou de mélange correspondant à la formule générale (1) suivante : 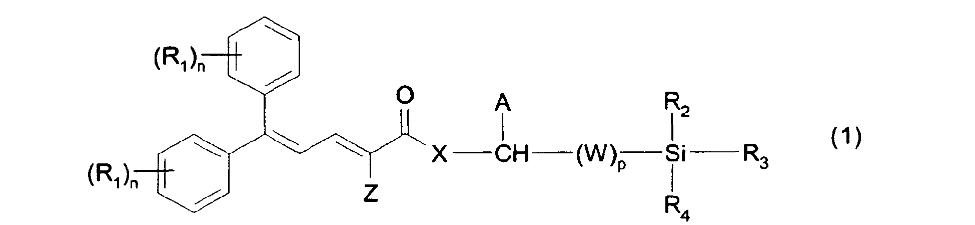 dans laquelle :
- les radicaux R₁ , égaux ou différents désignent un radical hydroxyle ; un radical alkyle en C₁-C₁₀, linéaire ou ramifié, saturé ou insaturé ; un radical alcoxy en C₁-C₁₀, linéaire ou ramifié ; ou bien 2 groupes R₁ adjacents pouvant former ensemble un cycle alkylène dioxy en C₁-C₃ ;
- les radicaux R₂, R₃ et R₄ identiques ou différents, représentent un radical alkyle en C₁-C₁₀, linéaire ou ramifié, saturé ou insaturé, pouvant comporter un ou plusieurs atomes d'halogène ; un radical phényle,
- p est égal à 0 ou 1.
- A désigne hydrogène ; un radical alkyle en C₁-C₈, linéaire ou ramifié, saturé ou insaturé; un radical phényle ; le groupe Si(CH₃)₃, sous réserve que lorsque A est Si(CH₃)₃, alors p = 0 et R₂, R₃ et R₄ sont méthyle,
- n est un nombre entier allant de 0 à 2,
- W est un radical alkylène en C₁-C₈ linéaire ou ramifié, saturé ou insaturé, éventuellement substitué par un radical hydroxyle,
- Z désigne hydrogène ; -(C=O)OR₅, -(C=O)R₆, -(C=O)NR₇R₈ -SO₂R₉ , -CN ou
- (C=O)XCHA(W)ₚSiR₂R₃R₄ ,
- le radical R₅ désigne hydrogène ; un radical alkyle en C₁-C₂₀, linéaire ou ramifié, saturé
ou insaturé,
- le radical R₆ designe un radical alkyle en C₁-C₂₀, linéaire ou ramifié, éventuellement cyclique ; un aryle en C₆-C₁₂,
- les radicaux R₇, R₈, identiques ou différents représentent hydrogène ; un alkyle en C₁-C₂₀, linéaire ou ramifié,
- X désigne -O- ou -NR₇-,
- le radical R₉ désigne un radical alkyle en C₁-C₂₀, linéaire ou ramifié ; un aryle en C₆-C₁₂.

2. Méthyltrialkylsilane à fonction 4,4-diarylbutadiène selon la revendication 1, présentant au moins l'une, et encore plus préférentiellement l'ensemble des caractéristiques suivantes :
- Z = -(C=O)OR₅ , -CN ou (C=O)XCHA(W)pSiR₂R₃R₄,
- R₅ est méthyle ou éthyle,
- A est H,
- R₂ à R₄ désignent alkyle en C₁-C₄ et plus préférentiellement méthyle,
- n est 0,
- W est un radical alkylène en C₁-C₂.

3. Méthyltrialkylsilane à fonction 4,4-diarylbutadiène selon la revendication 1 ou 2 choisi dans le groupe constitué des composés (A), (B) et (C) suivants :

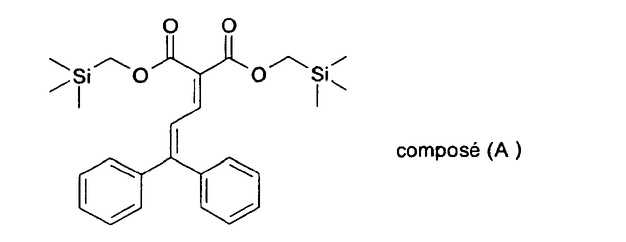

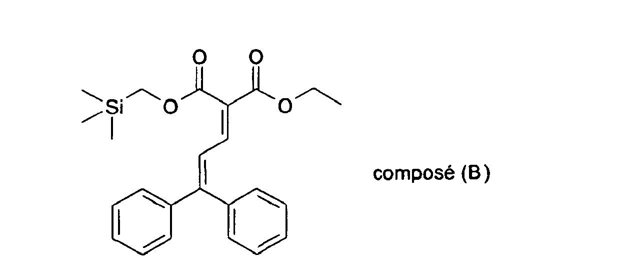

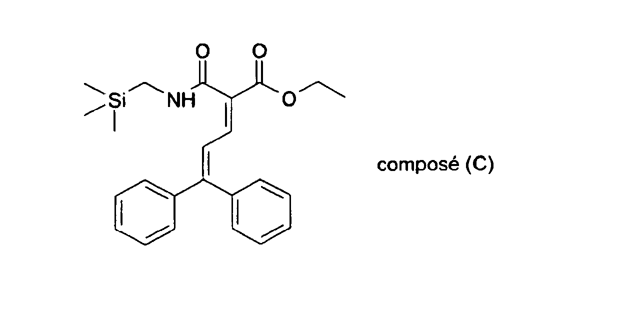

4. Méthyltrialkylsilane à fonction 4,4-diarylbutadiène de formule (1) selon la revendication 1 ou 2, où Z désigne -CN.

5. Méthyltrialkylsilane à fonction 4,4-diarylbutadiène de formule (1) selon la revendication 4 présentant au moins l'une, et encore plus préférentiellement l'ensemble des caractéristiques suivantes :
- A est H,
- R₂ à R₄ désignent alkyle en C₁-C₄ et plus préférentiellement méthyle,
- n est 0,
- p est 0 ou 1,
- W est un radical alkylène en C₁-C₂

6. Méthyltrialkylsilane à fonction 4,4-diarylbutadiène selon la revendication 4 ou 5 choisi dans le groupe constitué par les composés (D), (E), (F) et (G) suivants :

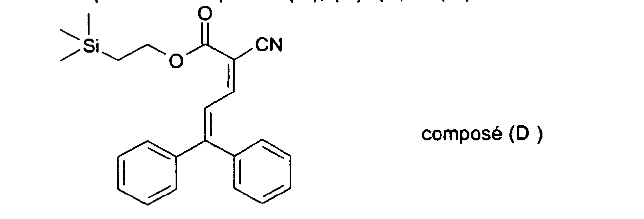

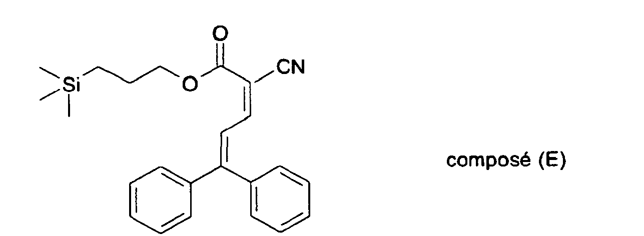

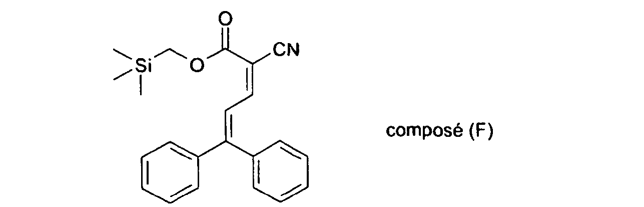

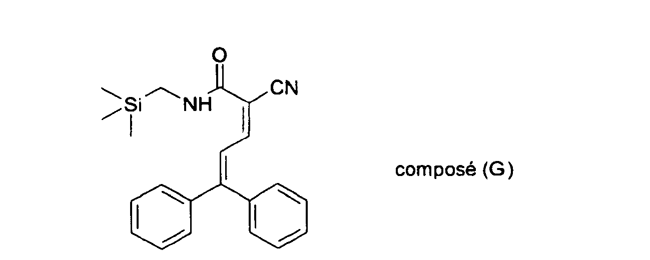

7. Utilisation d'un méthyltrialkylsilane à fonction 4,4-diarylbutadiène de formule (1) tel que défini dans l'une quelconque des revendications 1 à 6 dans la préparation une composition cosmétique ou dermatologique comme agent filtrant les radiations UV de longueurs d'onde allant de 320 à 400 nm.

8. Composition cosmétique ou dermatologique, destinée à la photoprotection des matières kératiniques, **caractérisée par le fait qu'**elle contient dans un support cosmétiquement acceptable au moins un composé méthyltrialkylsilane à fonction 4,4-diarylbutadiène répondant à la formule (1) tel que défini dans l'une quelconque des revendications 1 à 6.

9. Composition selon la revendication 8, **caractérisée par le fait que** le composé de formule (1) est présent dans la composition à une teneur allant de 0,01 à 20 % en poids, par rapport au poids total de la composition, de préférence allant de 0,1 % à 10 % en poids, par rapport au poids total de la composition.

10. Composition selon l'une quelconque des revendications 1 à 9, **caractérisée par le fait qu'**elle comprend en outre un ou plusieurs filtres organiques ou inorganiques complémentaires actifs dans l'UV-A et/ou UV-B.

11. Composition selon la revendication 10, **caractérisée par le fait que** lesdits filtres organiques complémentaires sont choisis les anthranilates ; les dérivés cinnamiques ; les dérivés de dibenzoylméthane ; les dérivés salicyliques, les dérivés du camphre ; les dérivés de triazine ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène ; les 4,4-diarylbutadiènes non-siliciés et leurs mélanges.

12. Composition selon la revendication 11, **caractérisée par le fait que** lesdits filtres organiques complémentaires sont choisis parmi
Ethylhexyl Salicylate,
Ethylhexyl Methoxycinnamate
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
2,4,6-tris-(4'-amino benzalmalonate de diisobutyle)-s-triazine
Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine et leurs mélanges.

13. Composition selon la revendication 10, **caractérisée par le fait que** les filtres complémentaires inorganiques sont des pigments ou des nanopigments d'oxydes métalliques, enrobés ou non.

14. Composition selon la revendication 13, **caractérisée par le fait que** lesdits pigments
ou nanopigments sont choisis parmi les oxydes de titane, de zinc, de fer, de zirconium, de cérium et leurs mélanges, enrobés ou non.

15. Composition selon l'une quelconque des revendications 7 à 14, **caractérisée par le fait qu'**elle comprend en outre au moins un agent de bronzage et/ou de brunissage artificiel de la peau.

16. Composition selon l'une quelconque des revendications 7 à 15, **caractérisée par le fait qu'**elle comprend en outre au moins un adjuvant choisi parmi les corps gras, les solvants organiques, les épaississants ioniques ou non ioniques, les adoucissants, les humectants, les antioxydants, les hydratants, les desquamants, les agents anti-radicalaires, les agents antipollution, les antibactériens, les agents anti-inflammatoires, les dépigmentants, les pro-pigmentants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les pigments, les polymères, les propulseurs, les agents alcalinisants ou acidifiants

17. Composition selon l'une quelconque des revendications 7 à 16, **caractérisée par le fait qu'**il s'agit d'une composition protectrice de l'épiderme humain ou d'une composition antisolaire et qu'elle se présente sous forme d'une dispersion vésiculaire non ionique, d'une émulsion, d'une crème, d'un lait, d'un gel, d'un gel crème, d'une suspension, d'une dispersion, d'une huile, d'une poudre, d'un bâtonnet solide, d'une mousse ou d'un spray.

18. Composition selon l'une quelconque des revendications 7 à 16, **caractérisée par le fait qu'**il s'agit d'une composition de maquillage des cils, des sourcils, des ongles ou de la peau et qu'elle se présente sous forme solide ou pâteuse, anhydre ou aqueuse, d'une émulsion, d'une suspension ou d'une dispersion.

19. Composition selon l'une quelconque des revendications 7 à 16, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

20. Utilisation d'au moins un composé silanique de formule (1) tel que défini dans l'une quelconque des revendications 1 à 6 dans une composition cosmétique comme agent de contrôle de la variation de la couleur de la peau due aux radiations UV de longueur d'onde allant de 320 à 400 nm.

21. Utilisation d'au moins un composé silanique de formule (1) tel que défini dans l'une quelconque des revendications 1 à 6 comme agent photostabilisant de polymères synthétiques ou de verres.

## Claims

1. Methyltrialkylsilane containing a 4,4-diarylbutadiene function, in isolated form or in the form of a mixture, corresponding to general formula (1) below: 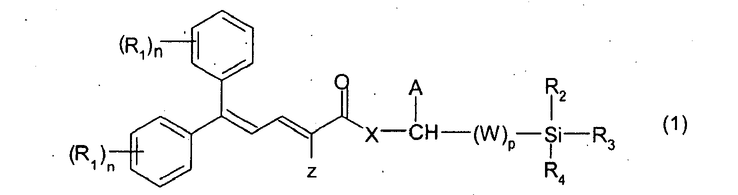 in which:
- the radicals R₁, which may be identical or different, denote a hydroxyl radical; a saturated or unsaturated, linear or branched C₁-C₁₀ alkyl radical; a linear or branched C₁-C₁₀ alkoxy radical; or else two adjacent groups R₁ together possibly forming a C₁-C₃ alkylenedioxy ring;
- the radicals R₂, R₃ and R₄, which are identical or different, represent a saturated or unsaturated, linear or branched C₁-C₁₀ alkyl radical which can contain one or more halogen atoms; or a phenyl radical,
- p is 0 or 1,
- A denotes hydrogen; a saturated or unsaturated, linear or branched C₁-C₈ alkyl radical; a phenyl radical; or the group Si(CH₃)₃, with the proviso that, if A is Si (CH₃)₃ , then p = 0 and R₂, R₃ and R₄ are methyl,
- n is an integer ranging from 0 to 2,
- W is a saturated or unsaturated, linear or branched C₁-C₈ alkylene radical optionally substituted by a hydroxyl radical,
- Z denotes hydrogen; - (C=O) OR₅ , - (C=O)R₆ , -(C=O)NR₇R₈,
- SO₂R₉, -CN Or - (C=O) XCHA (W) pSiR₂R₃R₄,
- the radical R₅ denotes hydrogen; or a saturated or unsaturated, linear or branched C₁-C₂₀ alkyl radical,
- the radical R₆ denotes a linear or branched, optionally cyclic, C₁-C₂₀ alkyl radical; or a C₆-C₁₂ aryl,
- the radicals R₇ and R₈, which are identical or different, represent hydrogen; or a linear or branched C₁-C₂₀ alkyl,
- X denotes -O- or -NR₇-, and
- the radical R₉ denotes a linear or branched C₁-C₂₀ alkyl radical; or a C₆-C₁₂ aryl.

2. Methyltrialkylsilane containing a 4,4-diarylbutadiene function according to Claim 1, satisfying at least one, and even more preferably all, of the following characteristics:
- Z = (C=O) OR₅, -CN or (C=O) XCHA (W) pSiR₂R₃R₄,
- R₅ is methyl or ethyl,
- A is H,
- R₂ to R₄ denote C₁-C₄ alkyl and more preferably methyl,
- n is 0, and
- W is a C₁-C₂ alkylene radical.

3. Methyltrialkylsilane containing a 4,4-diarylbutadiene function according to Claim 1 or 2, chosen from the group consisting of compounds (A), (B) and (C) below:

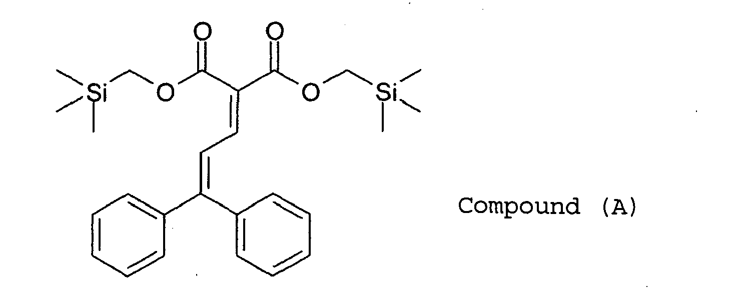

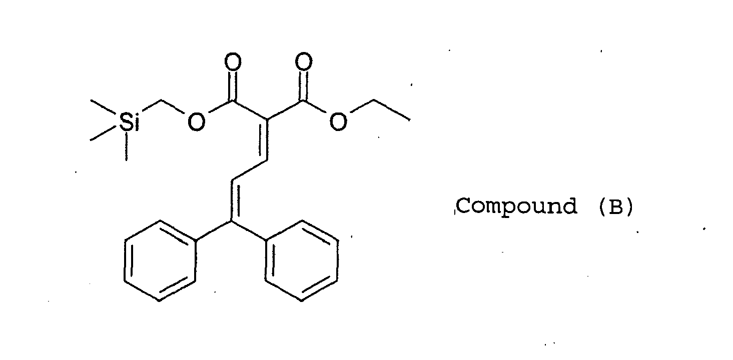

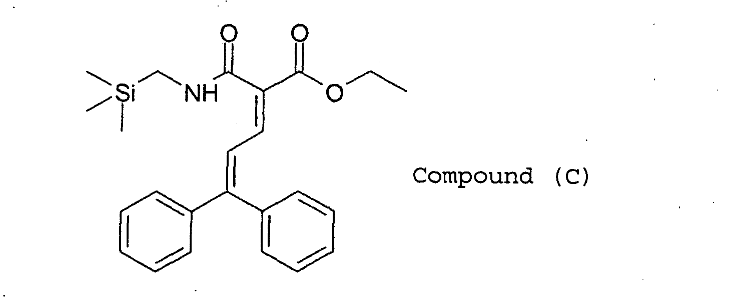

4. Methyltrialkylsilane containing a 4,4-diarylbutadiene function of formula (1) according to Claim 1 or 2, in which Z denotes -CN.

5. Methyltrialkylsilane containing a 4,4-diarylbutadiene function of formula (1) according to Claim 4, satisfying, at least one, and even more preferably all, of the following characteristics:
- A is H,
- R₂ to R₄ denote C₁-C₄ alkyl and more preferably methyl,
- n is 0,
- p is 0 or 1, and
- W is a C₁-C₂ alkylene radical.

6. Methyltrialkylsilane containing a 4,4-diarylbutadiene function according to Claim 4 or 5, chosen from the group consisting of compounds (D), (E), (F) and (G) below:

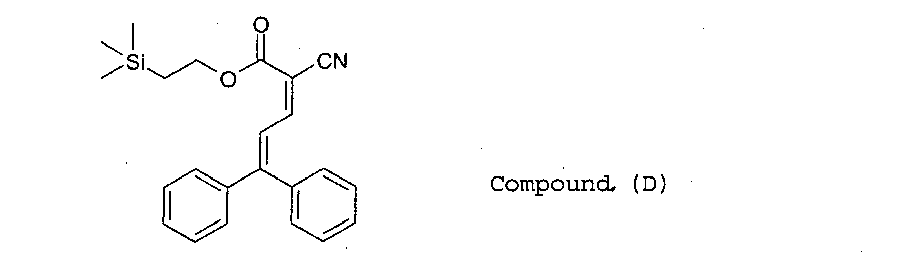

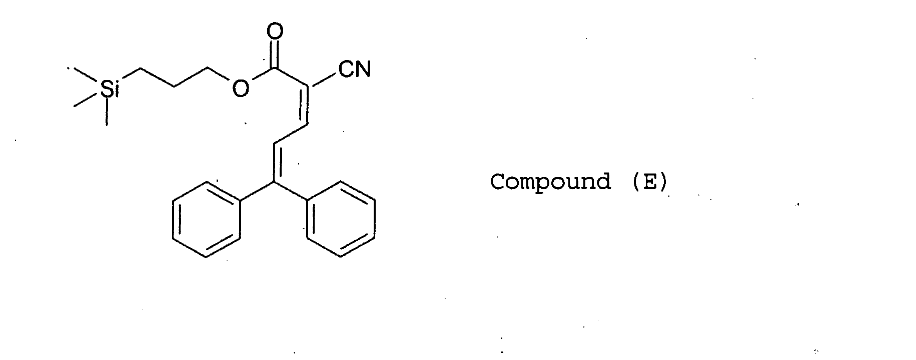

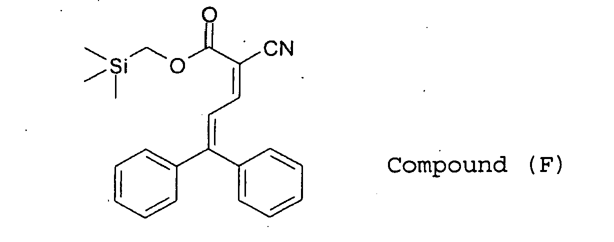

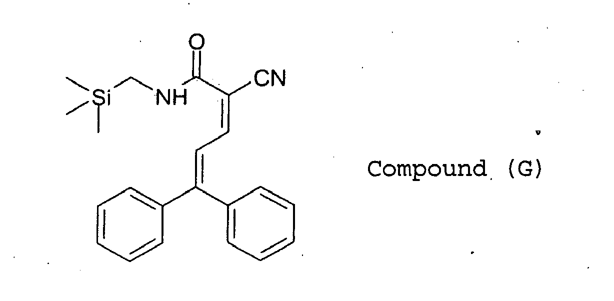

7. Use of a methyltrialkylsilane containing a 4,4-diarylbutadiene function of formula (1) as defined in any one of Claims 1 to 6, in the preparation of a cosmetic or dermatological composition, as an agent for screening out UV radiation with wavelengths ranging from 320 to 400 nm.

8. Cosmetic or dermatological composition for photoprotecting keratin materials, **characterized in that** it contains, in a cosmetically acceptable support, at least one methyltrialkylsilane compound containing a 4,4-diarylbutadiene function corresponding to formula (1) as defined in any one of Claims 1 to 6.

9. Composition according to Claim 8, **characterized in that** the compound of formula (1) is present in the composition in a content ranging from 0.01% to 20% by weight relative to the total weight of the composition, and preferably ranging from 0.1% to 10% by weight relative to the total weight of the composition.

10. Composition according to any one of Claims 1 to 9, **characterized in that** it also comprises one or more additional UV-A-active and/or UV-B-active organic or mineral screening agents.

11. Composition according to Claim 10, **characterized in that** the said additional organic screening agents are chosen from anthranilates; cinnamic derivatives; dibenzoylmethane derivatives; salicylic derivatives; camphor derivatives; triazine derivatives; benzophenone derivatives; β,β-diphenylacrylate derivatives; benzotriazole derivatives; benzalmalonate derivatives; benzimidazole derivatives; imidazolines; bis-benzazolyl derivatives; p-aminobenzoic acid (PABA) derivatives; methylenebis(hydroxyphenylbenzotriazole) derivatives; benzoxazole derivatives; screening polymers and screening silicones; α-alkylstyrene-based dimers; non-siliceous 4,4-diarylbutadienes, and mixtures thereof.

12. Composition according to Claim 11, **characterized in that** the said additional, organic screening agents are chosen from:
Ethylhexyl salicylate,
Ethylhexyl methoxycinnamate,
Butyl methoxydibenzoylmethane,
Octocrylene,
Phenylbenzimidazolesulfonic acid,
Benzophenone-3,
Benzophenone-4,
Benzophenone-5,
n-Hexyl 2-(4-diethylamino-2-hydroxybenzoyl)benzoate,
4-Methylbenzylidenecamphor,
Terephthalylidenedicamphorsulfonic acid,
Disodium phenyldibenzimidazoletetrasulfonate,
2,4,6-Tris(diisobutyl 4'-aminobenzalmalonate)-s-triazine,
Anisotriazine,
Ethylhexyltriazone,
Diethylhexylbutamidotriazone,
Methylenebis(benzotriazolyl)tetramethylbutylphenol,
Drometrizole trisiloxane,
Polysilicone-15,
1,1-Dicarboxy(2,2'-dimethylpropyl)-4,4-diphenylbutadiene,
2,4-Bis[5-1(dimethylpropyl)benzoxazol-2-yl(4-phenyl)imino]-6-(2-ethylhexyl)imino-1,3,5-triazine and mixtures thereof.

13. Composition according to Claim 10, **characterized in that** the additional mineral screening agents are coated or uncoated metal oxide pigment's or nanopigments.

14. Composition according to Claim 13, **characterized in that** the said pigments or nanopigments are chosen from coated or uncoated titanium oxide, zinc oxide, iron oxide, zirconium oxide and cerium oxide, and mixtures thereof.

15. Composition according to any one of Claims 7 to 14, **characterized in that** it also comprises at least one agent for artificially tanning and/or browning the skin.

16. Composition according to any one of Claims 7 to 15, **characterized in that** it also comprises at least one adjuvant chosen from fatty substances, organic solvents, ionic or nonionic thickeners, softeners, humectants, antioxidants, moisturizers, desquamating agents, free-radical scavengers, antipollution agents, antibacterial agents, anti-inflammatory agents, depigmenting agents, propigmenting agents, opacifiers, stabilizers, emollients, silicones, antifoams, insect repellents, fragrances, preserving agents, anionic, cationic, nonionic, zwitterionic or amphoteric surfactants, substance P antagonists, substance CGRP antagonists, fillers, pigments, polymers, propellants, acidifying or basifying agents.

17. Composition according to any one of Claims 7 to 16, **characterized in that** it is a composition for protecting the human epidermis or an antisun composition and **in that** it is in the form of a nonionic vesicular dispersion, an emulsion, a cream, a milk, a gel, a cream-gel, a suspension, a dispersion, an oil, a powder, a solid tube, a mousse or a spray.

18. Composition according to any one of Claims 7 to 16, **characterized in that** it is a composition for making up the eyelashes, the eyebrows, the nails or the skin and **in that** it is in solid or pasty, anhydrous or aqueous form or in the form of an emulsion, a suspension or a dispersion.

19. Composition according to any one of Claims 7 to 16, **characterized in that** it is a composition for protecting the hair against ultraviolet rays and **in that** it is in the form of a shampoo, a lotion, a gel, an emulsion or a nonionic vesicular dispersion.

20. Use of at least one silane compound of formula (1) as defined in any one of Claims 1 to 6, in a cosmetic composition as an agent for controlling the variation in the colour of the skin caused by UV radiation with wavelengths ranging from 320 to 400 nm.

21. Use of at least one silane compound of formula (1) as defined in any one of Claims 1 to 6, as an agent for photostabilizing synthetic polymers or lenses.

## Patentansprüche

1. Methyltrialkylsilan mit 4,4-Diarylbutadienfunktion der folgenden allgemeinen Formel (1) in isolierter Form oder als Gemisch: 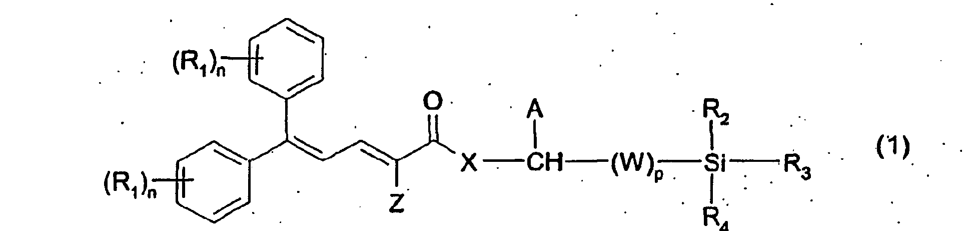 in der Formel:
- die Gruppen R₁, die gleich oder verschieden sind, bedeuten eine Hydroxygruppe; eine geradkettige oder verzweigte, gesättigte oder ungesättigte C₁₋₁₀-Alkylgruppe; eine geradkettige oder verzweigte C₁₋₁₀-Alkoxygruppe; oder zwei benachbarte Gruppen R₁ können gemeinsam einen C₁₋₃-Alkylendioxyring bilden;
- die Gruppen R₂, R₃ und R₄, die gleich oder verschieden sind, bedeuten eine geradkettige oder verzweigte, gesättigte oder ungesättigte C₁₋₁₀-Alkylgruppe, die ein oder mehrere Halogenatome enthalten kann; eine Phenylgruppe;
- p ist 0 oder 1;
- A bedeutet Wasserstoff; eine geradkettige oder verzweigte, gesättigte oder ungesättigte C₁₋₈-Alkylgruppe; eine Phenylgruppe; die Gruppe Si(CH₃)₃, mit der Maßgabe, dass p= 0 und R₂, R₃ und R₄ bedeuten Methyl, wenn A Si(CH₃)₃ ist;
- n ist eine ganze Zahl im Bereich von 0 bis 2;
- W bedeutet eine geradkettige oder verzweigte, gesättigte oder ungesättigte C₁₋₈-Alkylengruppe, die gegebenenfalls mit einer Hydroxygruppe substituiert ist;
- Z bedeutet Wasserstoff; -(C=O)OR₅, -(C=O)R₆, -(C=O)NR₇R₈,
- SO₂R₉, -CN oder -(C=O)XCHA(W)ₚSiR₂R₃R₄,
- die Gruppe R₅ bedeutet Wasserstoff; eine geradkettige oder verzweigte, gesättigte oder ungesättigte C₁₋₂₀-Alkylgruppe;
- die Gruppe R₆ bedeutet eine geradkettige oder verzweigte C₁₋₂₀-Alkylgruppe, die gegebenenfalls cyclisch ist; eine C₆-₁₂-Arylgruppe;
- die Gruppen R₇ und R₈, die gleich oder verschieden sind, bedeuten Wasserstoff, eine geradkettige oder verzweigte C₁₋₂₀-Alkylgruppe;
- X ist -O- oder -NR₇-;
- die Gruppe R₉ bedeutet eine geradkettige oder verzweigte C₁₋₂₀-Alkylgruppe; eine C₆-₁₂-Arylgruppe.

2. Methyltrialkylsilan mit 4,4-Diarylbutadienfunktion nach Anspruch 1, das mindestens ein und noch bevorzugter alle folgenden Merkmale aufweist:
- Z = -(C=O)OR₅, -CN oder (C=O)XCHA(W)ₚSiR₂R₃R₄,
- R₅ bedeutet Methyl oder Ethyl,
- A ist H,
- R₂ bis R₄ bedeuten C₁₋₄-Alkyl und vorzugsweise Methyl,
- n ist 0,
- W bedeutet eine C₁₋₂-Alkylengruppe.

3. Methyltrialkylsilan mit 4,4-Diarylbutadienfunktion nach Anspruch 1 oder 2, das unter den folgenden Verbindungen (A), (B) und (C) ausgewählt ist:

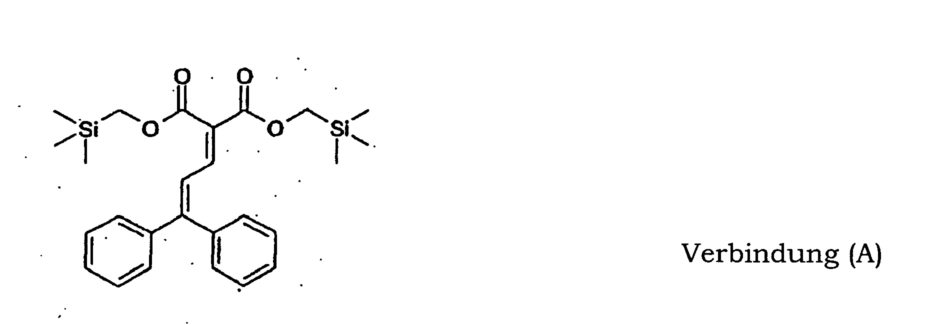

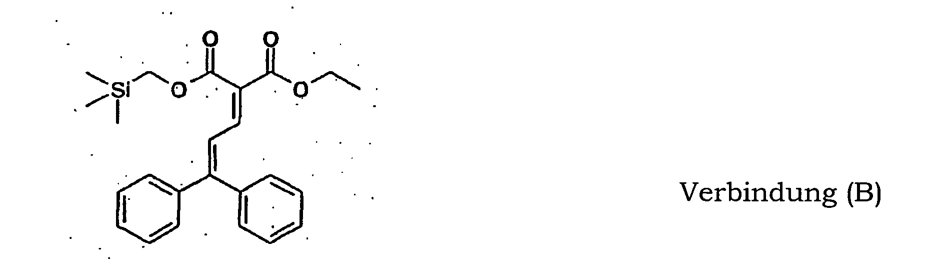

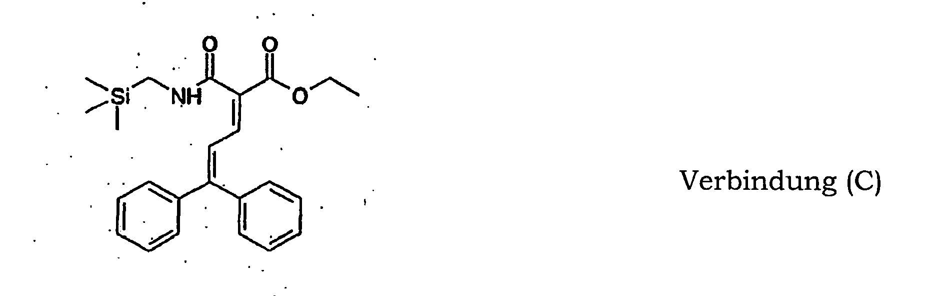

4. Methyltrialkylsilan mit 4,4-Diarylbutadienfunktion der Formel (1) nach Anspruch 1 oder 2, wobei Z -CN bedeutet.

5. Methyltrialkylsilan mit 4,4-Diarylbutadienfunktion der Formel (1) nach Anspruch 4, das mindestens ein und noch bevorzugter alle folgenden Merkmale aufweist:
- A ist H,
- R₂ bis R₄ bedeuten C₁₋₄-Alkyl und vorzugsweise Methyl,
- n ist 0,
- p ist 0 oder 1,
- W bedeutet eine C₁₋₂-Alkylengruppe.

6. Methyltrialkylsilan mit 4,4-Diarylbutadienfunktion der Formel (1) nach Anspruch 4 oder 5, das unter den folgenden Verbindungen (D), (E), (F) und (G) ausgewählt ist:

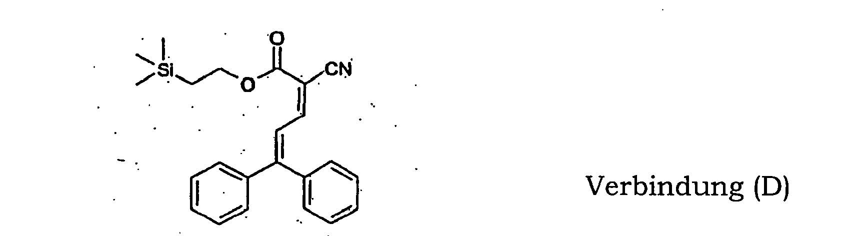

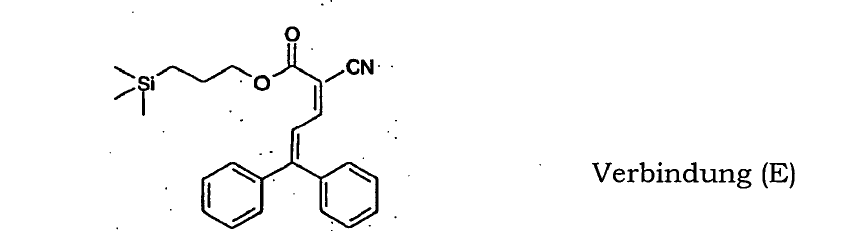

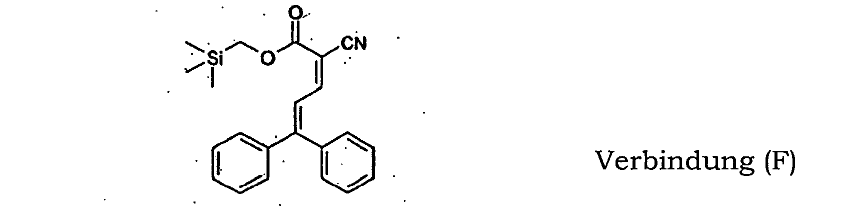

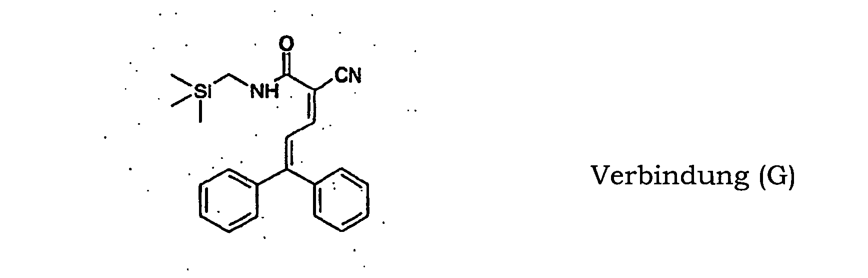

7. Verwendung eines Methyltrialkylsilans mit 4,4-Diarylbutadienfunktion der Formel (1) nach einem der Ansprüche 1 bis 6 für die Herstellung einer kosmetischen oder dermatologischen Zusammensetzung als Stoff, der UV-Strahlung im Wellenlängenbereich von 320 bis 400 nm filtert.

8. Kosmetische oder dermatologische Zusammensetzung, die für den Lichtschutz von Keratinsubstanzen vorgesehen ist, **dadurch gekennzeichnet, dass** sie in einem kosmetisch akzeptablen Träger mindestens ein Methyltrialkylsilan mit 4,4-Diarylbutadienfunktion der Formel (1) nach einem der Ansprüche 1 bis 6 enthält.

9. Zusammensetzung nach Anspruch 8, **dadurch gekennzeichnet, dass** die Verbindung der Formel (1) in der Zusammensetzung in einer Menge von 0,01 bis 20 Gew.-%, bezogen auf das Gesamtgewicht der Zusammensetzung, und vorzugsweise 0,1 bis 10 Gew.-%, bezogen auf das Gesamtgewicht der Zusammensetzung, enthalten ist.

10. Zusammensetzung nach einem der Ansprüche 1 bis 9, **dadurch gekennzeichnet, dass** sie ferner ein oder mehrere ergänzende, im UV-A-Bereich und/oder UV-B-Bereich wirksame organische oder anorganische Filter enthält.

11. Zusammensetzung nach Anspruch 10, **dadurch gekennzeichnet, dass** die ergänzenden organischen Filter ausgewählt sind unter den Anthranilaten; Zimtsäurederivaten; Dibenzoylmethanderivaten; Salicylsäurederivaten; Campherderivaten; Triazinderivaten; Benzophenonderivaten; β, β-Diphenylacrylatderivaten, Benzotriazolderivaten; Benzalmalonatderivaten; Benzimidazolderivaten; Imidazolinen; Bisbenzazolylderivaten; Derivaten von p-Aminobenzoesäure (PABA); Methylen-bis(hydroxyphenylbenzotriazol)derivaten; Benzoxazolderivaten; Filterpolymeren und Filtersiliconen; von α-Alkylstyrol abgeleiteten Dimeren; nicht siliciumhaltigen 4,4-Diarylbutadienen und deren Gemischen.

12. Zusammensetzung nach Anspruch 11, **dadurch gekennzeichnet, dass** die zusätzlichen organischen Filter ausgewählt sind unter:
Ethylhexyl Salicylate,
Ethylhexyl Methoxycinnamate,
Butyl Methoxydibenzoylmethane,
Octocrylene,
Phenylbenzimidazole Sulfonic Acid,
Benzophenone-3,
Benzophenone-4,
Benzophenone-5,
n-Hexyl-2-(4-diethylamino-2-hydroxybenzoyl)benzoat,
4-Methylbenzylidene Camphor,
Terephthalylidene Dicamphor Sulfonic Acid,
Disodium Phenyl Dibenzimidazole Tetra-sulfonate,
2,4,6-Tris(diisobutyl-4'-aminobenzalmalonat)-s-triazin, Anisotriazine,
Ethylhexyl Triazone,
Diethylhexyl Butamido Triazone,
Methylene bis-Benzotriazolyl Tetramethylbutylphenol,
Drometrizole Trisiloxane,
Polysilicone-15,
1,1-Dicarboxy-(2,2'-dimethyl-propyl)-4,4-diphenylbutadien,
2,4-Bis[5-1 (dimethylpropyl)benzoxazol-2-yl-(4-phenyl)imino]-6-(2-ethylhexyl)-imino-1,3,5-triazin und deren Gemischen.

13. Zusammensetzung nach Anspruch 10, **dadurch gekennzeichnet, dass** die ergänzenden anorganischen Filter beschichtete oder unbeschichtete Pigmente oder Nanopigmente von Metalloxiden sind.

14. Zusammensetzung nach Anspruch 13, **dadurch gekennzeichnet, dass** die Pigmente oder Nanopigmente unter den Oxiden von Titan, Zink, Eisen, Zirconium und Cer und deren Gemischen ausgewählt sind, wobei sie umhüllt oder nicht umhüllt sind.

15. Zusammensetzung nach einem der Ansprüche 7 bis 14, **dadurch gekennzeichnet, dass** sie ferner mindestens ein Bräunungsmittel und/ oder Mittel zur künstlichen Braunfärbung der Haut enthält.

16. Zusammensetzung nach einem der Ansprüche 7 bis 15, **dadurch gekennzeichnet, dass** sie ferner mindestens einen Zusatzstoff enthält, der unter den Fettsubstanzen, organischen Lösungsmitteln, ionischen oder nichtionischen Verdickungsmitteln, Weichmachern, Feuchthaltemitteln, Antioxidantien, Moisturizern, Abschuppungsmitteln, Radikalfängern für freie Radikale, Antipollutionswirkstoffen, antibakteriellen Wirkstoffen, entzündungshemmenden Wirkstoffen, Depigmentierungsmitteln, pigmentierungsfördernden Wirkstoffen, Trübungsmitteln, Stabilisatoren, Emollientien, Siliconen, Schaumverhütungsmitteln, Insekten abwehrenden Wirkstoffen, Parfums, Konservierungsmitteln, anionischen, kationischen, nichtionischen, zwitterionischen oder amphoteren grenzflächenaktiven Stoffen, Substanz P-Antagonisten, Substanz CGRP-Antagonisten, Füllstoffen, Pigmenten, Polymeren, Treibmitteln und Ansäuerungsmitteln oder Alkalisierungsmitteln ausgewählt ist.

17. Zusammensetzung nach einem der Ansprüche 7 bis 16, **dadurch gekennzeichnet, dass** es sich um eine Zusammensetzung für den Schutz der menschlichen Epidermis oder ein Sonnenschutzmittel handelt und **dadurch**, dass sie in der Form einer nichtionischen Vesikeldispersion, als Emulsion, Creme, Milch, Gel, Gelcreme, Suspension, Dispersion, Öl, Pulver, fester Stift, Schaum oder Spray vorliegt.

18. Zusammensetzung nach einem der Ansprüche 7 bis 16, **dadurch gekennzeichnet, dass** es sich um ein Schminkprodukt für die Wimpern, die Augenbrauen, die Nägel oder die Haut handelt, und **dadurch**, dass sie in fester oder pastöser, wasserfreier oder wässriger Form, in der Form einer Emulsion, Suspension oder Dispersion vorliegt.

19. Zusammensetzung nach einem der Ansprüche 7 bis 16, **dadurch gekennzeichnet, dass** es sich um eine Zusammensetzung zum Schutz der Haare gegen UV-Strahlung handelt und **dadurch**, dass sie als Haarwaschmittel, Lotion, Gel, Emulsion oder nichtionische Vesikeldispersion vorliegt.

20. Verwendung mindestens eines Silans der Formel (1) nach einem der Ansprüche 1 bis 6 in einer kosmetischen Zusammensetzung als Stoff für die Kontrolle der Farbänderung der Haut, die durch UV-Strahlung im Wellenlängenbereich von 320 bis 400 nm hervorgerufen wird.

21. Verwendung eines Silans der Formel (I) nach einem der Ansprüche 1 bis 6 als Stoff zur Photostabilisierung von synthetischen Polymeren oder Gläsern.
